(19) Europäisches Patentamt
European Patent Office
Office européen des brevets

(11) Numéro de publication : **0 484 247 A1**

(12) **DEMANDE DE BREVET EUROPEEN**

(21) Numéro de dépôt : **91402930.1**

(22) Date de dépôt : **31.10.91**

(51) Int. Cl.$^5$ : **A63B 26/00, A61B 5/103**

(30) Priorité : **31.10.90 FR 9013571**

(43) Date de publication de la demande :
**06.05.92 Bulletin 92/19**

(84) Etats contractants désignés :
**BE DE ES GB IT NL**

(71) Demandeur : **Pez, René**
**11 Chaussée de La Muette**
**F-75016 Paris (FR)**

(72) Inventeur : **Pez, René**
**11 Chaussée de la Muette**
**F-75016 Paris (FR)**
Inventeur : **Sejournet, Jérôme**
**18 Rue Gallieni**
**F-92100 Boulogne (FR)**

(74) Mandataire : **Bruder, Michel**
**Cabinet Michel Bruder Conseil en Brevets 10,**
**rue de la Pépinière**
**F-75008 Paris (FR)**

(54) **Plateau d'équilibre destiné à la rééducation.**

(57)    La présente invention concerne un plateau d'équilibre du type utilisé dans le domaine de la rééducation, monté pivotant autour d'au moins un axe horizontal (13).
    Ce plateau est caractérisé en ce qu'il comporte des moyens capteurs (15) aptes à transformer la position angulaire relative du plateau (1) par rapport à l'horizontale en signal électrique, des moyens redresseurs (20) du signal électrique fourni par les moyens capteurs (15), des moyens intégrateurs (24) dans le temps du signal électrique produit par les moyens redresseurs (20), et fournissant une valeur intégrée, des moyens d'affichage (28) de la valeur intégrée fournie par les moyens intégrateurs (24).

FIG. 3

EP 0 484 247 A1

La présente invention concerne un plateau d'équilibre du type utilisé dans le domaine de la rééducation.

On connaît des plateaux de ce type qui sont articulés autour d'un ou de deux axes perpendiculaires et horizontaux destinés à lui conférer un ou deux degrés de liberté. Le patient vient prendre appui par l'une de ses jambes sur le plateau et essaie ensuite de se maintenir en équilibre sur celui-ci. La recherche de l'équilibre réalisée par le patient peut ainsi répondre à deux objectifs.

Un premier objectif consiste à déterminer, de façon analytique, l'état de ses muscles ou de ses articulations en mettant en évidence, au moyen dudit plateau d'équilibre, certaines tendances à un déséquilibre dans un sens et ou dans un autre, c'est-à-dire d'avant en arrière, ou vers la gauche ou la droite.

Un second objectif consiste à faire travailler certains muscles ou certaines articulations trouvées déficientes, de façon progressive et contrôlée par un kinésithérapeute, de façon à améliorer l'état de ceux-ci.

Les dispositifs de l'art antérieur nécessitent ainsi, tant en ce qui concerne la détection de certaines anomalies que le travail de muscles ou d'articulations, la présence permanente d'un kinésithérapeute, tout d'abord pour détecter un déséquilibre éventuel du patient, et ensuite pour contrôler l'évolution de la thérapie réalisée par celui-ci.

La présente invention a ainsi pour but un procédé et un dispositif permettant de détecter un éventuel déséquilibre se manifestant chez un patient, d'indiquer le sens de déséquilibre et de quantifier ce dernier.

L'invention a également pour but un moyen permettant de déterminer le moment où, au cours de la séance de travail, les performances du patient sont maximales et/ou minimales.

La présente invention a ainsi pour objet un plateau d'équilibre du type utilisé dans le domaine de la rééducation, monté pivotant autour d'au moins un axe horizontal caractérisé en ce qu'il comporte des moyens capteurs aptes à transformer la position angulaire relative du plateau par rapport à l'horizontale en signal électrique, des moyens redresseurs et des moyens d'intégration dans le temps dudit signal électrique produits par les moyens capteurs, des moyens d'affichage de la valeur intégrée obtenue.

Dans une première variante intéressante du plateau d'équilibre suivant l'invention, le capteur est constitué d'un potentiomètre dont le corps et l'axe de commande sont respectivement liés aux deux parties en mouvement, à savoir le plateau d'équilibre proprement dit et son support, ce potentiomètre fournissant une tension variable, fonction de la position angulaire du plateau, par rapport à l'horizontale, cette tension étant maximale lorsque le plateau est basculé complètement dans un sens, et minimale lorsque le plateau est basculé complètement en sens inverse.

Dans une autre variante intéressante de l'invention la valeur de la tension intégrée dans les moyens intégrateurs est, périodiquement, divisée par le temps écoulé depuis le début de la séance, de façon à fournir au patient une valeur moyenne de la performance qu'il a réalisée depuis le début de celle-ci.

Dans une autre variante de l'invention on stocke périodiquement la performance la meilleure réalisée par le patient et, si cette valeur est meilleure que la meilleure des valeurs précédentes, la nouvelle valeur prend la place de l'ancienne. La même opération peut également être effectuée en ce qui concerne la performance la moins bonne réalisée par le patient. Ces valeurs peuvent également faire l'objet d'un affichage.

On décrira ci-après, à titre d'exemple non limitatif, plusieurs formes d'exécution de la présente invention, en référence au dessin annexé sur lequel :

La figure 1 est une vue en élévation d'un plateau d'équilibre suivant l'invention.

La figure 2 est une vue en coupe partielle et transversale d'un mode de mise en oeuvre d'un capteur utilisé dans le dispositif suivant l'invention.

La figure 3 est un schéma bloc représentant un mode de mise en oeuvre du dispositif suivant l'invention.

La figure 4 est un schéma bloc représentant une première variante du mode de mise en oeuvre représenté sur la figure 3.

La figure 5 est une représentation schématique d'un plateau d'équilibre suivant l'invention.

La figure 6 est un graphique représentant la variation de la tension aux bornes du capteur utilisé dans le mode de mise en oeuvre illustré par la figure 2, en fonction de l'angle d'inclinaison du plateau d'équilibre par rapport à l'horizontale.

Les figures 7 et 8 représentent un exemple de signal de tension en fonction du temps respectivement aux bornes du capteur et en sortie du circuit redresseur.

La figure 9 est un schéma bloc d'une variante de mise en oeuvre de l'invention.

La figure 10 représente un exemple de signal une fois celui-ci numérisé par un convertisseur analogique/numérique mis en oeuvre dans le mode de réalisation de la figure 9.

Les figures 11 et 12 sont des schémas blocs de deux variantes de réalisation de l'invention.

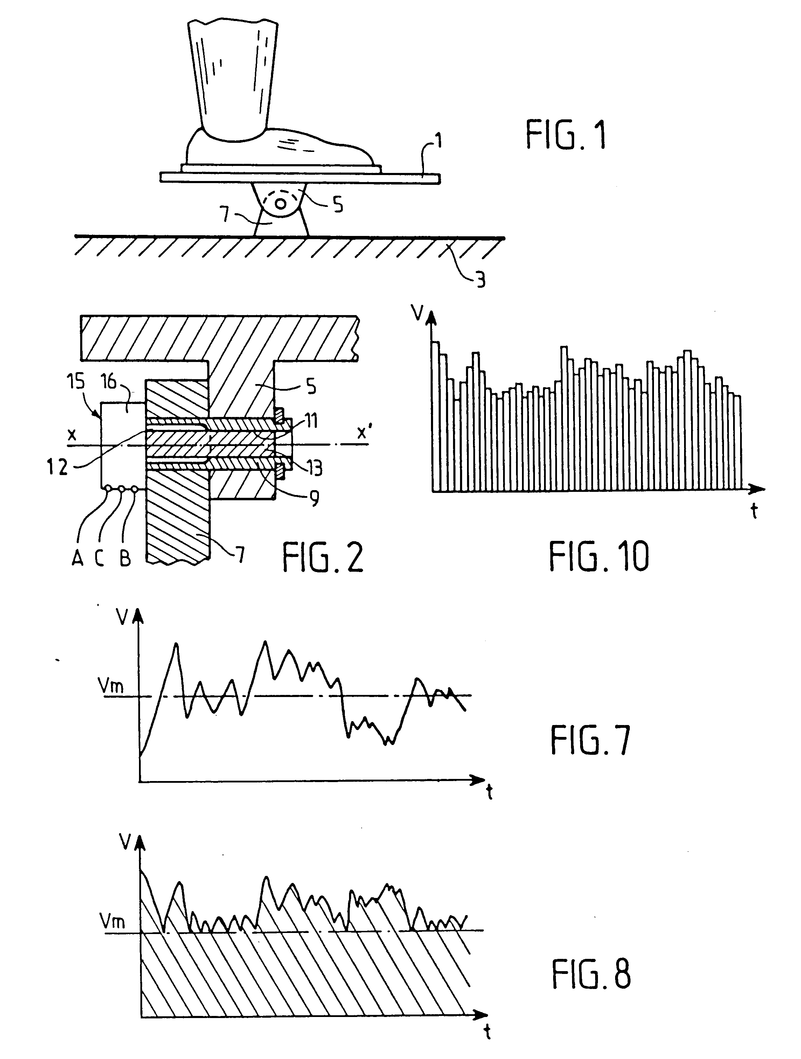

Sur les figures 1 et 2 un plateau d'équilibre de type classique se compose essentiellement d'un plateau 1 proprement dit comportant, au voisinage de chacune de ses extrémités, une patte verticale 5 lui permettant d'être articulé autour d'un axe horizontal xx', sur un support 7 en contact avec le sol 3. Le support 7 et la patte d'articulation 5 de la planche d'équilibre 1 sont traversés par un axe de rotation 9 percé d'un trou axial 11, dont une extrémité se termine par un chambrage 12 de plus grand diamètre. Le trou axial 11 est traversé par une tige de commande 13 d'un potentiomètre 15 qui est solidarisé du tube 9, par exemple par emmanchement à force. Le boîtier 16 du potentiomètre est, quant à lui, fixé sur la paroi verticale du support 7. De cette façon toute rotation du plateau d'équilibre 1, autour de l'axe 9, provoque la rotation de la tige de commande 13 du potentiomètre par rapport à son boîtier 16. Le potentiomètre 15 constitue ainsi un capteur de rotation, puisque toute rotation du plateau 1 par rapport à son support 7 entraîne la rotation de la tige de commande 13 du potentiomètre par rapport à son boîtier 16, cette rotation se traduisant par une différence de potentiel v entre les bornes AC du potentiomètre 15, correspondant à une extrémité de la résistance de celui-ci et de son curseur, qui est variable avec la position angulaire du plateau 1.

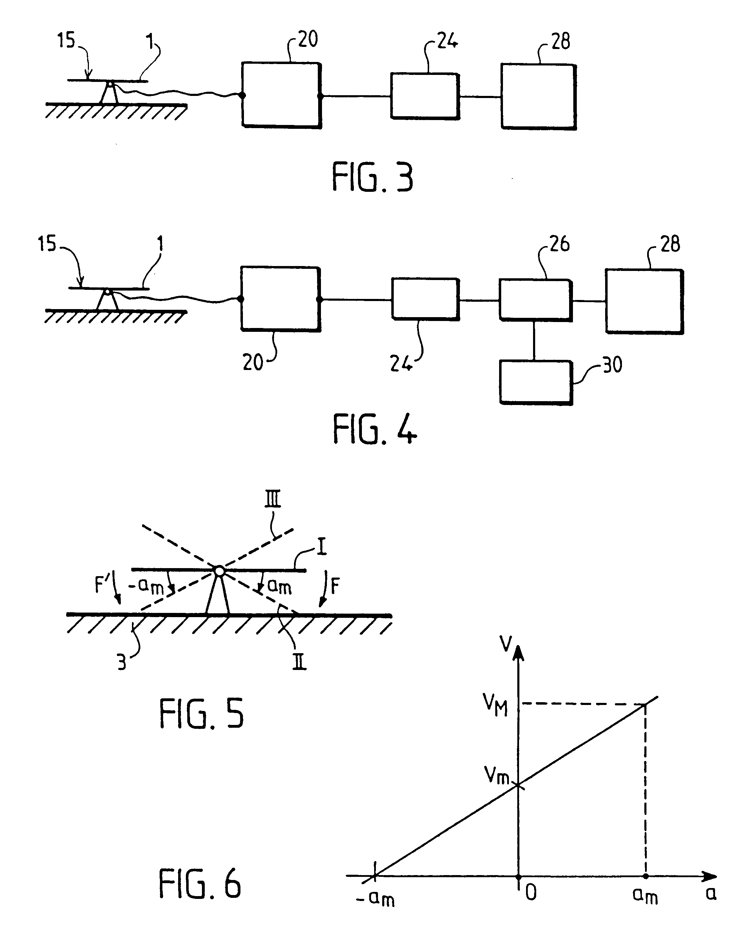

Le graphique de la figure 5 représente ainsi la tension v disponible entre les bornes A et C du potentiomètre 15 en fonction de l'angle $\underline{a}$ formé par le plateau 1 avec l'horizontale. Ainsi, comme représenté sur les figures 5 et 6, lorsque le plateau 1 se trouve en position parfaitement horizontale, (position médiane I), c'est-à-dire lorsque l'angle $\underline{a}$ est égal à 0, la tension aux bornes A et C du potentiomètre 15 est de $v_m$. Lorsque, à partir de cette position médiane I le patient fait pivoter le plateau 1 dans le sens des aiguilles d'une montre, à savoir la direction de la flèche F, l'angle $\underline{a}$ augmente, ainsi que la tension v aux bornes du potentiomètre 15, pour atteindre une valeur $v_M$ lorsque le plateau vient en butée avec le sol 3, ce qui correspond à un angle $\underline{a}$ maximal égal à $a_m$ (position II de la figure 5). A l'inverse, lorsque l'utilisateur, partant de la position médiane I fait pivoter le plateau 1 dans le sens inverse des aiguilles d'une montre, à savoir la direction de la flèche F', celui-ci forme avec l'horizontale un angle -a, et le potentiomètre 15 fournit entre ses bornes A et C une tension v inférieure à la tension $v_m$, pour atteindre, lorsque l'autre extrémité du plateau 1 vient en contact avec le sol 3 (ce qui correspond à la position III de la figure 4), une tension voisine de 0.

Bien entendu, suivant la présente invention, il est possible d'utiliser tout autre capteur de rotation permettant, de préférence, d'obtenir entre ses bornes une tension variable fonction de l'inclinaison $\underline{a}$ du plateau 1 avec l'horizontale.

Le signal ainsi disponible entre les bornes A et C du capteur est ensuite traité par des moyens électroniques, objets de plusieurs variantes de mise en oeuvre de l'invention.

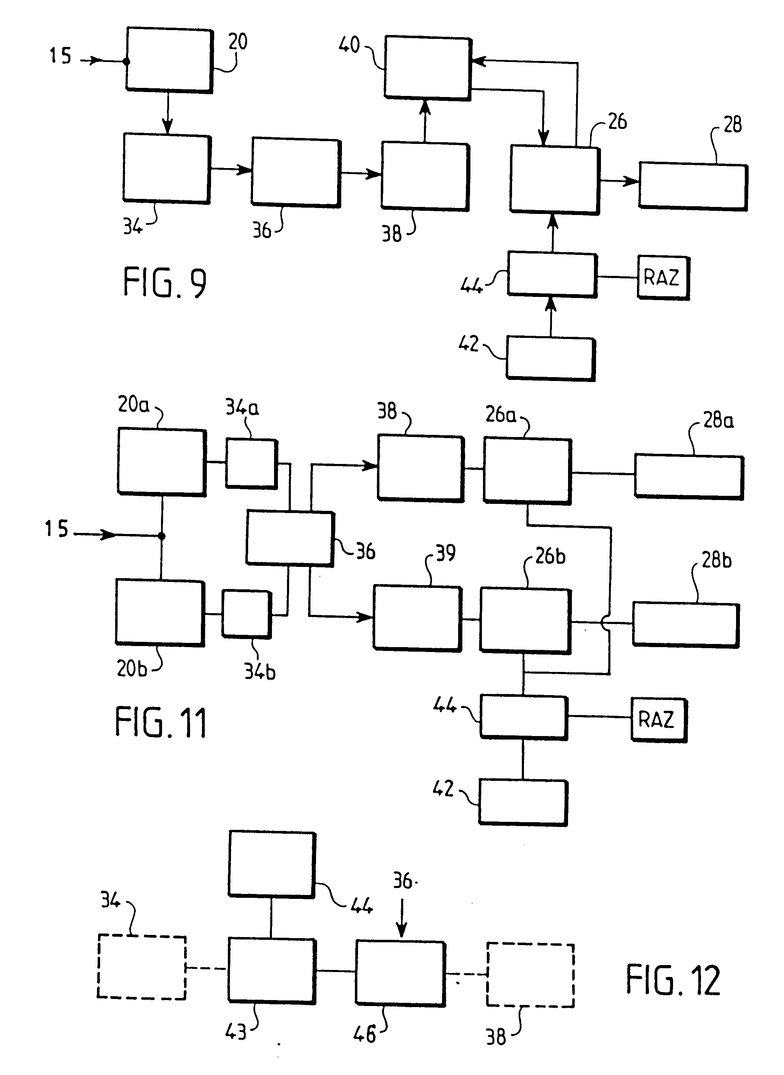

Dans le mode de mise en oeuvre représenté sur la figure 3 le signal en sortie du capteur 15 est acheminé vers des moyens redresseurs 20 qui, à partir d'un signal variant en fonction du temps, tel que celui représenté sur la figure 7 et possédant des parties de courbe se situant au-dessus de la tension de position médiane $v_m$, correspondant à la position horizontale du plateau, et des portions de courbes se situant sous cette partie horizontale, fournit une variation de tension en fonction du temps se situant intégralement au-dessus de la valeur moyenne $V_m$ représentée sur la figure 6. Le signal en sortie du redresseur est fourni à un circuit intégrateur 24, qui fournit, comme montré sur la figure 8, un signal proportionnel à la surface comprise entre le signal v=f (t) et l'axe des temps. Cette valeur intégrée du signal est représentative des oscillations effectuées par le patient au cours de sa séance de travail. En effet, au cours de celle-ci, plus le patient réalisera d'oscillations de part et d'autre de la zone d'équilibre horizontale du plateau 1, plus ladite surface fournie par le circuit intégrateur 24 sera importante.

Le circuit intégrateur 24 est suivi d'un circuit afficheur 28, qui fournit en permanence au patient, et de façon quasiment instantanée, un nombre représentatif de ladite surface et donc de l'importance des oscillations qu'il a réalisées, ce qui lui permet de juger de la qualité de sa performance.

Afin d'améliorer la signification du nombre fourni au patient on peut, comme représenté sur la figure 4, interposer entre la sortie du circuit intégrateur 24 et l'entrée du circuit afficheur 28 un circuit diviseur 26, connecté à un circuit 30 de mesure du temps, ce circuit diviseur 26 divisant ladite valeur intégrée par le temps écoulé depuis le début de la séance de travail. Le quotient affiché est ainsi plus représentatif de la performance réalisée par le patient, puisqu'il est indépendant de la durée de l'exercice réalisé par celui-ci, ce qui lui permet de réaliser une comparaison instantanée avec des mesures ultérieures.

Dans une autre variante de l'invention représentée sur la figure 9, le signal en sortie du capteur 15 est fourni à un circuit redresseur 20 dont la sortie est reliée à un circuit convertisseur analogique/numérique 34, c'est-à-dire un circuit transformant les signaux analogiques en sortie du redresseur 20 en une série de signaux numériques échantillonnés (figure 10). Ces signaux numériques sont stockés dans une mémoire 36 puis additionnés dans un additionneur 38 et le résultat de cette addition est stocké dans une seconde mémoire 40. D'autre part un circuit horloge 42 alimente un circuit compteur 44, ce circuit compteur 44, ainsi que la sortie du second circuit mémoire 40, alimentant un circuit diviseur 26 permettant d'obtenir une valeur représentant la somme des oscillations numérisées divisées par le temps qui s'est écoulé depuis le début de la séance de travail. Comme précédemment le circuit diviseur 26 est relié à un circuit d'affichage 28 qui fournit donc à l'utilisateur un nombre représentant la valeur moyenne de sa performance.

Dans une autre variante de l'invention représentée de façon schématique sur la figure 11, le signal en provenance du capteur 15 aboutit à l'entrée de deux circuits redresseurs, à savoir un circuit redresseur 20a redressant la partie du signal situé au-dessus de la valeur moyenne $v_m$ et un redresseur 20b redressant la partie du signal situé au-dessous de cette même valeur moyenne $v_m$. Ces valeurs sont respectivement numérisées dans deux circuits convertisseurs analogique/numérique respectivement 34a et 34b et stockées dans une mémoire 36. En sortie de cette mémoire 36 les valeurs correspondantes numérisées sont respectivement fournies à un additionneur 38 et à un soustracteur 39 puis, après passage dans deux circuits diviseurs respectifs 26a et 26b, dans lesquels elles sont divisées par le temps qui s'est écoulé depuis le début de la mesure, ces valeurs sont respectivement fournies à deux afficheurs 28a et 28b.

Comme précédemment les valeurs affichées ayant traversé le circuit additionneur 38 représentent la moyenne globale de la totalité des oscillations du plateau, c'est-à-dire qu'elle prend en compte aussi bien les oscillations produites dans un sens que celles produites dans l'autre.

La valeur fournie par l'afficheur 28b représente, quant à elle, la différence des signaux produits au-dessus de la valeur médiane $v_m$ et des signaux produits en dessous de celle-ci, ramenée par le diviseur 26b à l'état de moyenne. Cette valeur est représentative de la tendance du patient à produire des oscillations d'un côté déterminé, c'est-à-dire en avant ou en arrière, ou bien sur la gauche ou sur la droite.

La présente invention permet donc de détecter chez un patient un défaut de comportement ou une tendance, et est en mesure de quantifier celui-ci, ce qui lui permet de mesurer, au fur et à mesure de la pratique des exercices, l'évolution de ce défaut et de cette même tendance.

Bien entendu, la saisie des données, en sortie du capteur 15 peut être réalisée par des moyens automatisés tels que par exemple un microprocesseur ou un ordinateur. Ces derniers pourront ainsi enregistrer les mesures, au fur et à mesure des séances successives, de façon à réaliser par exemple des histogrammes illustrant l'évolution des performances réalisées par le patient.

La présente invention permet également de fournir au patient la meilleure et/ou la plus mauvaise des performances qu'il aura réalisée pendant un temps donné déterminé. Pour cela, comme représenté sur la figure 12, on pourra disposer, en sortie de circuit analogique/numérique 34 de la figure 9, un circuit additionneur 43, relié à des moyens de comptage de temps 44 additionnant les signaux numériques produits pendant un temps donné, la valeur obtenue $V_o$ étant fournie à des moyens comparateurs 46 en relation avec la mémoire 36. Les moyens comparateurs 46 compareront la valeur obtenue avec respectivement la plus mauvaise valeur et la meilleure valeur respectivement stockée dans la mémoire, et remplaceront les valeurs stockées par la valeur courante $V_o$ suivant que celle-ci est respectivement inférieure ou supérieure aux valeurs stockées. Bien entendu ces valeurs, c'est-à-dire la meilleure et la plus mauvaise, peuvent être affichées, et l'utilisateur dispose ainsi d'une indication supplémentaire permettant de mesurer ses performances.

Bien entendu, bien que les circuits représentés se rapportent à une planche d'équilibre à un degré de liberté, il est possible, comme mentionné précédemment, d'en prévoir au moins un second, et dans ce cas, les circuits électroniques utilisés pourraient être soit doublés soit multiplexés.

## Revendications

1.- Plateau d'équilibre du type utilisé dans le domaine de la rééducation, monté pivotant autour d'au moins un axe horizontal (13) caractérisé en ce qu'il comporte des moyens capteurs (15) aptes à transformer la position angulaire relative du plateau (1) par rapport à l'horizontale en signal électrique, des moyens redresseurs (20,20a,20b) du signal électrique fourni par les moyens capteurs (15), des moyens intégrateurs dans le temps (24,34,38) du signal électrique produit par les moyens redresseurs (20,20a,20b), et fournissant une valeur intégrée, des moyens d'affichage (28,28a,28b) de la valeur intégrée fournie par les moyens intégrateurs (24,34,38).

2.- Plateau d'équilibre suivant la revendication 1 caractérisé en ce que les moyens intégrateurs sont constitués de moyens de conversion analogique/numérique (34) transformant les signaux analogiques fournis par les moyens redresseurs (20,20a,20b) en une série de signaux numériques, et de moyens additionneurs (38) destinés à faire la somme desdits signaux numériques de façon à intégrer la valeur du signal dans le temps.

3.- Plateau d'équilibre suivant l'une quelconque des revendications précédentes caractérisé en ce qu'il comporte des moyens de comptage du temps (42,44) et des moyens diviseurs (26) disposés en sortie des moyens intégrateurs (24,34,38) et divisant ladite valeur intégrée obtenue, par le temps écoulé depuis le début d'une mesure, fourni par les moyens de comptage du temps.

4.- Plateau d'équilibre suivant la revendication 1 caractérisé en ce qu'il comporte des moyens mémoire (36) aptes à stocker ladite valeur intégrée.

5.- Plateau d'équilibre suivant la revendication 1 caractérisé en ce que les moyens redresseurs sont constitués de deux circuits redresseurs (20a,20b) redressant respectivement la partie du signal fourni par les moyens capteurs (15) se situant au-dessus et au-dessous d'une tension médiane $v_m$, correspondant à la valeur de la tension fournie par les moyens capteurs (15) lorsque le plateau (1) se trouve en position horizontale, ces valeurs étant respectivement communiquées à des moyens intégrateurs (34a,34b), respectivement reliés à un circuit additionneur (38) et à un circuit soustracteur (39) réalisant respectivement l'addition et la soustraction des susdites valeurs, les circuits additionneur (38) et soustracteur (39) étant respectivement reliés à des moyens d'affichage (28a,28b).

6.- Plateau d'équilibre suivant la revendication 5 caractérisé en ce qu'il comporte des moyens mémoire (36) aptes à stocker les valeurs intégrées des valeurs de tension respectivement situées au-dessus et au-dessous de la valeur médiane de tension $v_m$.

7.- Plateau d'équilibre suivant la revendication 6 caractérisé en ce que les circuits additionneur (38) et soustracteur (39) sont respectivement reliés à des circuits diviseurs (26a,26b) eux-mêmes en communication avec des circuits de comptage du temps, les circuits diviseurs (26a,26b) divisant les valeurs respectives fournies par les circuits additionneur (38) et soustracteur (39), par le temps écoulé depuis le début d'une mesure, les circuits diviseurs respectifs (26a,26b) étant reliés à des afficheurs (28a,28b).

8.- Plateau d'équilibre suivant l'une quelconque des revendications précédentes caractérisé en ce qu'un circuit additionneur (43) est disposé en sortie du circuit convertisseur analogique/numérique (34), ce circuit étant relié à des moyens de comptage du temps (44), le circuit additionneur (43) additionnant les signaux numériques produits pendant un temps donné, une valeur obtenue courante ($V_o$) étant fournie à des moyens comparateurs (46) en relation avec une mémoire (36), ces moyens comparateurs (46) comparant la valeur obtenue courante ($V_o$) avec respectivement une valeur la plus mauvaise, et une valeur la meilleure, stockées dans la mémoire (36), et remplaçant ces valeurs stockées, par la valeur courante ($V_o$), suivant que celle-ci est respectivement moins bonne ou meilleure que les valeurs stockées.

FIG. 1

FIG. 2

FIG. 10

FIG. 7

FIG. 8

FIG. 3

FIG. 4

FIG. 5

FIG. 6

FIG. 9

FIG. 11

FIG. 12

Office européen
des brevets

## RAPPORT DE RECHERCHE EUROPEENNE

Numero de la demande

EP    91 40 2930

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | FR-A-2 472 929 (M KURTZ)<br>* page 1, ligne 28 - page 2, ligne 6 *<br>* page 3, ligne 21 - ligne 37 *<br>* page 5, ligne 17 - page 6, ligne 10 *<br>* page 6, ligne 26 - page 7, ligne 8 *<br>* figures 1-3 *<br>--- | 1,2,4-6 | A63B26/00<br>A61B5/103 |
| A | US-A-3 712 294 (J MULLER)<br>--- | 1 | |
| A | US-A-3 906 931 (Y TEREKHOV)<br>--- | 1 | |
| A | WO-A-8 808 275 (H HUBERTI)<br>--- | 2-8 | |
| A | US-A-4 893 808 (D MCINTYRE)<br>----- | 1 | |

DOMAINES TECHNIQUES RECHERCHES (Int. Cl.5 )

A63B
A61B

Le présent rapport a été établi pour toutes les revendications

| Lieu de la recherche | Date d'achèvement de la recherche | Examinateur |
|---|---|---|
| LA HAYE | 05 FEVRIER 1992 | VEREECKE A. |

EPO FORM 1503 03.82 (P0402)